Europäisches Patentamt

European Patent Office

Office européen des brevets

(11) Numéro de publication: **0 359 888**
**A1**

(12) **DEMANDE DE BREVET EUROPEEN**

(21) Numéro de dépôt: **88600011.6**

(51) Int. Cl.5: **A61N 1/22**

(22) Date de dépôt: **20.09.88**

(43) Date de publication de la demande:
**28.03.90 Bulletin 90/13**

(84) Etats contractants désignés:
**AT BE CH DE ES FR GB GR IT LI LU NL SE**

(71) Demandeur: **Papadopoulos, Georges**
**48, Ermou Street**
**546 23 Thessaloniki(GR)**

(72) Inventeur: **Papadopoulos, Georges**
**48, Ermou Street**
**546 23 Thessaloniki(GR)**

(74) Mandataire: **Varotsos, Ioannis**
**45 Solonos Street**
**106 72 Athens(GR)**

(54) **Ceintures (lombes, nuque, genoux) d'action dynamique pour l'utilisation de l'energie bioélectrique produite par des électrodes.**

(57) Ceinture lombaire (No4), collier cervical (No5) ou genouillère pour le traitement de la partie respective souffrante sur lesquels sont fixés, selon les nécessités du traitement deux ou plusieurs lames métalliques rondes ou carrées avec une forme conique, trapezoïdale (No1) ou plate, d'une épaisseur identique. L'une de ces lames contient principalement du cuivre et l'autre du zinc en grande proportion.

Lorsqu'on applique la ceinture, le collier ou la genouillère sur la région affectée , il se produit, à travers la peau,un courant diadermique de basse tension,par la dissemblance des composants des lames, lorsqu'elles entrent en contact avec la peau humide de la partie souffrante.

Cette ceinture est formée par un tissu rectangulaire, à trame serrée, sur lequel sont fixées les lames (No2) au moyen d'un second tissu plastifié, ayant les mêmes dimensions que la ceinture,percé d'ouvertures plus petites que les dimensions des lames (No3). De cette manière, la partie centrale des lames apparaît libre (à découvert).

N° 2

**CEINTURES (lombes, nuque, genoux) d'action dynamique pour l'utilisation de l'énergie bioélectrique produite par des électrodes.**

## A. DESCRIPTION:

La ceinture lombaire, le collier cervical, la genouillère ou n'importe quel autre matériel de bandage s'appliquant à la région affectée comprend:

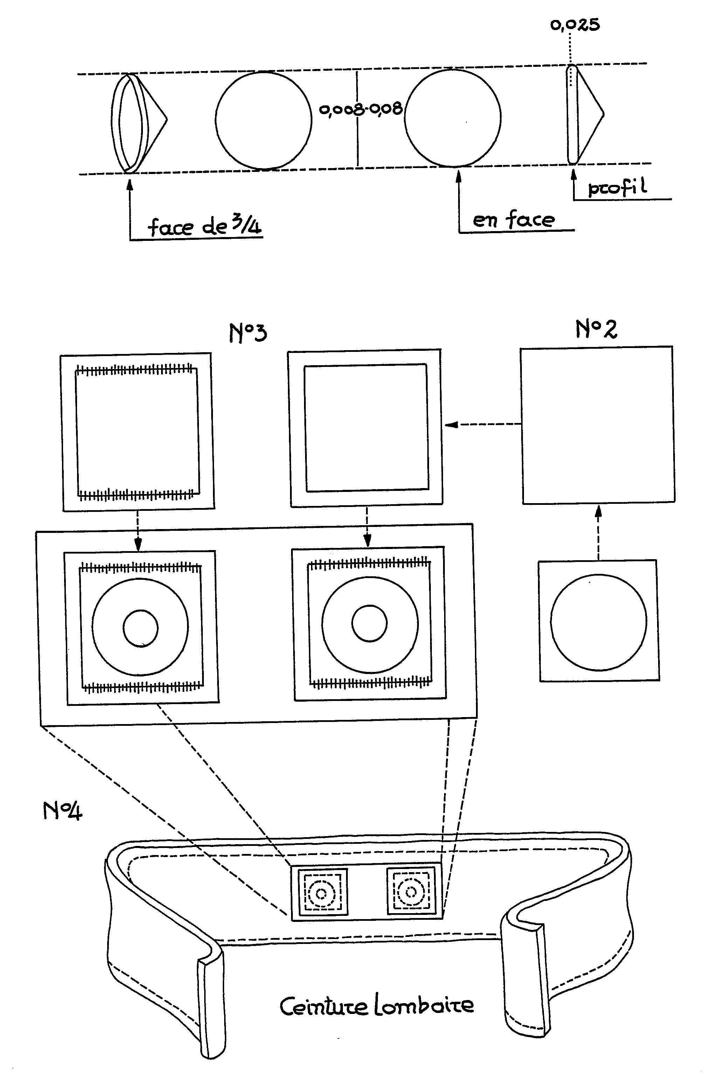

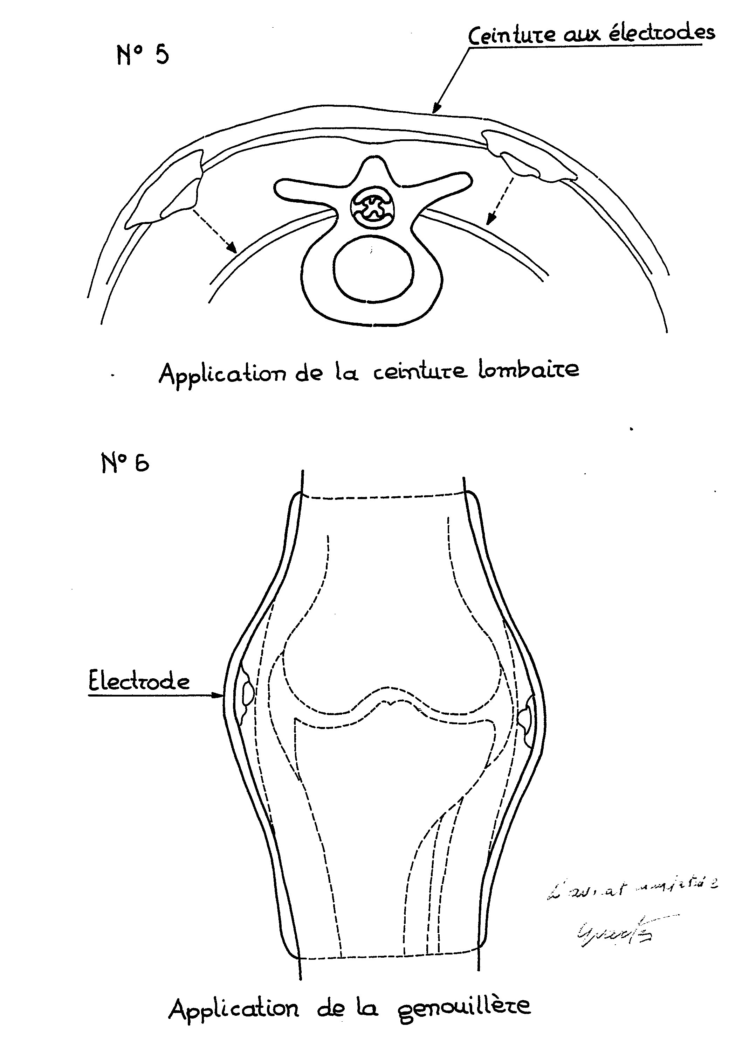

(a) deux (ou plusieurs) lames métalliques périphériques de composition différente et de formes variées, rondes ou carrées de préférence d'épaisseurs diverseset de formes conique, trapezoïdale ou même plate. L'une de ces lames contient principalement du cuivre et l'autre du zinc en grande proportion. Le pourtout, l'épaisseur ainsi que la forme et les dimensions des deux lames sont absolument pareils. Quand elles sont rondes, leur diamètre en principe est: 0,008 à 0,08m. et leur épaisseur de 0,025m. comme elles apparaissent sur la figure No1.-NOTE: Ces dimensions peuvent être modifiées selon les nécessités du traitement.

(b) un tissu rectangulaire, à trame serrée, sur lequel se fixent les lames métalliques (électrodes) décrites ci-dessus. Cette application s'effectue par l'application d'une seconde couche formée d'un tissu plastifié ayant les mêmes dimensions, percé d'ouvertures d'un diamètre inférieur aux dimensions des électrodes (lames métalliques). De cette manière, la partie centrale de ces la mes apparaît librement (à découvert). Cela apparaît clairement sur la figure No4. Nous pourrions dénommer l'élément décrit au paragraphe "b" cihaut "pièce de rechange" puisqu'il est possible de remplacer cette pièce par une nouvelle lorsqu'elle s'oxyde (quand elle devient inopérante) Observation importante: Si après deux ou trois jours le patient qui porte la ceinture (avec la "pièce de rechange") n'éprouve aucune amélioration vers sa guérison, il convient d'intervertir les pôles de la "pièce de rechange"

## B. UTILISATION-FONCTIONNEMENT

Nous fixons le tissu rectangulaire muni des deux electrodes soit par la surface arrière autocollante, soit au moyen de simples agrafes. L'application s'effectue sur la surface intérieure de la ceinture lombaire qui porte les électrodes. Les électrodes touchent ainsi la peau de la région affectée, de part et d'autre de la colonne vertébrale ou de l'articulation. La surface de la peau devra être humide et chaude et cela se réalise par la ceinture munie des électrodes. La pression sur la surface humide, chaude de la peau, des sommets des lames métalliques, qui agissent comme électrodes entraîne un mouvement divergent d'électrons qui, en conséquence, produit une différence de potentiel.

Le courant qui est produit varie entre 30 et 70 microvolts (2,3 - 875 microampères) selon le degré de transpiration du corps et la friction qui a lieu dans la région qui est soumise au traitement.

La forme de courant qui apparaît sur la surface fluorescente de l'appareil de mesurage est irrégulière et de forme ellipsoïde.

La partie rectangulaire du tissu muni d'électrodes est additionnelle et elle vient s'adapter à la ceinture lombaire ou au collier cervical. Lorsque les électrodes s'oxydent, ce tissu est remplacé par un autre qui est identique, à l'instar d'une "pièce de rechange". Ainsi, le patient ne supporte pas les frais d'achat d'une nouvelle ceinture, mais seulement d'une pièce de rechange munie d'électrodes. On estime que cet élément doit être remplacé après vingt jours d'usage.

## C. RESULTATS

A la suite de très longues études et selon une récente communication scientifique au Congrès International des Associations Orthopédistes qui s'est tenu du 8 au 11 Mai 1986, les résultats obtenus sont les suivantes:

Des applications ont été effectuées sur 1600 + 96 soit en tout sur 1696 patients de l'Hôpital Général "Hippocratio" et de l'Hôpital des Assurances Sociales de Thessalonique.

Résultats: 55,20 o/o bons
30,20 o/o moyens
14,60 o/o

Nous évaluons l'amélioration d'après la diminution ou la disparition des douleurs avec une recrudescence simultanée de l'activité du patient.

Observation: Cette statistique se réfère à des patients qui n'étaient pas soumis à un traitement pharmaceutique.

## D. APPLICATIONS.

Les ceintures plus haut décrites s'appliquent aux lombes, à la nuque, aux épaules ou à l'articulation du genou selon des modes de soutien différents. Elles s'adaptent, également, de manière autocollante, à toute partie du corps où apparaissent des douleurs névralgiques provoquées par des arthrites rhumatismales, des refroidissements ou des syndromes post-traumatiques (complications).

Elles s'appliquent en outre pour la tonicité des muscles abdominaux indirectement par l'excitation des racines qui correspondent aux nerfs des parois abdominales ayant fait l'objet d'une neurotomie.

Cependant les applications de la ceinture à action dynamique ne s'étend pas aux cas où des traitements chirurgicaux précis sont indiqués, ni non plus aux glissements du corps de vertèbres (Spondylolisthésis) ou de paraplégie. Mais il est possible que la ceinture influence indirectement la circulation artérielle des membres inférieurs en agissant sur les ganglions de la chaîne sympathique lombaire.

## Revendications

(1) Ceinture lombaire (No4) composée d'un tissu rectangulaire à trame serrée sur lequel sont fixées, à l'endroit où cette ceinture entrera en contact avec les lombes,deux (2) lames métalliques exactement identiques, rondes ou carrées de préférence (No2) avec une forme conique - trapezoïdale (No1) ou même plate, d'une même épaisseur et variant selon les nécessités thérapeutiques. L'une de ces lames contient principalement du cuivre et l'autre contient du zinc en grande proportion.
La fixation des lames s'opère par l'application d'une seconde couche formée par un tissu plastifié ayant les mêmes dimensions que le tissu de la ceinture. Le tissu plastifié est percé d'ouvertures d'un diamètre inférieur aux dimensions des lames métalliques (No3). Ainsi, la partie centrale des lames apparaît librement (à découvert). Cette ceinture, pour le traitement de lombalgies, se caractérise par l'application diadermique, à travers la peau, des courants de basse tension qui sont produits par les lames dont la composition est dissemblable, lorsqu'elles entrent en contact avec la peau humide du patient.

(2) Ceinture cervicale composée des mêmes éléments décrits plus haut sub No1. Ses dimensions comme celles des lames métalliques sont adéquates pour leur application sur la nuque (No5)

(3) Genouillère composée également des mêmes éléments que ceux décrits ci-haut sous le No1. Ses dimensions comme celles des lames métalliques sont adéquates pour le genou (No6).

(4) Ceinture pour lombes, pour la nuque et genouillère décrites plus haut sous les numéros 1. 2. 3. munies de lames en cuivre et en zinc pouvant être appliquées selon les nécessités thérapeutiques. Dans ce cas, le tissu plastifié décrit au paragraphe 1 ci-dessus comporte des ouvertures correspondant et s'adaptant aux lames métalliques.

0,025

0,008-0,08

profil

face de 3/4

en face

N°3

N°2

N°4

Ceinture Lombaire

N° 5

Ceinture aux électrodes

Application de la ceinture lombaire

N° 6

Electrode

Application de la genouillère

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.3) |
|---|---|---|---|
| X | GB-A-  2 187  (SHORT)(A.D. 1910) <br> * En entier * <br> --- | 1-4 | A 61 N   1/22 |
| A | GB-A-2 109 689  (MIDDLETON) <br> * En entier * <br> ----- | 1-4 | |

**DOMAINES TECHNIQUES RECHERCHES (Int. Cl.3)**

A 61 N

Le présent rapport a été établi pour toutes les revendications

| Lieu de la recherche | Date d'achèvement de la recherche | Examinateur |
|---|---|---|
| LA HAYE | 22-05-1989 | LEMERCIER D.L.L. |